# EUROPÄISCHE PATENTSCHRIFT

(11) **EP 2 321 241 B1**
(45) Veröffentlichungstag und Bekanntmachung des Hinweises auf die Patenterteilung: **19.09.2012**
(21) Anmeldenummer: 09781102.0
(22) Anmeldetag: 27.07.2009
(51) Int. Cl.: C07C 29/149, C07C 67/317, C07C 67/343, C07C 69/65, C07C 69/738

(54) **VERFAHREN ZUR HERSTELLUNG VON PHENYLALKAN-1-OLEN**
METHOD FOR PRODUCING PHENYLALKANE-1-OLS
PROCÉDÉ DE PRÉPARATION DE PHÉNYLALCANE-1-OLS

(30) Priorität: 01.08.2008 EP 08161643
(43) Veröffentlichungstag der Anmeldung: 18.05.2011
(73) Patentinhaber: BASF SE, 67056 Ludwigshafen (DE)
(72) Erfinder: WEIS, Martine, 68165 Mannheim (DE); BREUNINGER, Daniel, 67240 Bobenheim-Roxheim (DE); EBEL, Klaus, 68623 Lampertheim (DE); WINSEL, Harald, 67251 Freinsheim (DE)
(86) Internationale Anmeldenummer: PCT/EP2009/059638
(87) Internationale Veröffentlichungsnummer: WO 2010/012675

(56) Entgegenhaltungen:
- WO-A-2006/125026
- DE-C- 40 747
- L.A. SAUDAN ET AL.: "Dihydrogen Reduction of Carboxylic Esters to Alcohols under the Catalysis of Homogeneous Ruthenium Complexes: High Efficiency and Unprecedented Chemoselectivity" ANGEWANDTE CHEMIE, Bd. 119, 2007, Seiten 7617-7620, XP002545260 in der Anmeldung erwähnt

## Beschreibung

Die vorliegende Erfindung betrifft ein Verfahren zur Herstellung von Phenylalkan-1-olen über drei Stufen, wobei in der ersten Stufe eine Esterkondensation in Gegenwart von Alkali- oder Erdalkalialkoholaten durchgeführt wird.

Es besteht ein großer Bedarf an Phenylalkan-1-olen, die als Vorprodukt oder Zwischenprodukt für verschiedenste Anwendungen wie u.a. Riechstoffe, Pharmawirkstoffe oder Pflanzenschutzmittel verwendet werden. So ist beispielsweise 3-(3'-Trifluormethylphenyl)propan-1-ol (3-TFMPP) ein mögliches Vorprodukt bei der Synthese des Arzneimittels Cinacalcet (Mimpara®, Sensipar®), welches in der Therapie von sekundärem Hyperparathyreoidismus eingesetzt wird.

In WO 2006125026 werden verschiedene Routen zu Cinacalcet ausgehend von 3-(3'-Trifluormethylphenyl)propylamin, 3-(3'-Trifluormethylphenyl)propionaldehyd, 3-(3'-Trifluormethylphenyl)propionitril und 3-(3'-Trifluormethylphenyl)propan-1-ol (3-TFMPP) beschrieben. Die Synthese von Cinacalcet ausgehend von 3-TFMPP beinhaltet die Umwandlung der OH-Gruppe in eine gute Abgangsgruppe und gefolgt von der Umsetzung mit (R)-Naphthylethylamin in Gegenwart einer Base. Diese Route weist mehrere Vorteile gegenüber den anderen Synthesewegen auf. So wird beispielsweise der Einsatz von giftigen, kostspieligen und schwer handhabbaren Stoffen wie Ti(Oi-Pr)₄, NaBH₃CN, Oxalylchlorid oder Dimethylsulfoxid vermieden.

In diesem Zusammenhang werden in WO 2006125026 zwei unterschiedliche Synthesemöglichkeiten für den Alkohol, die beide von 1-Brom-3-(trifluormethyl)-benzol ausgehen, beschrieben. Der Aromat wird einer Heck-Kreuzkupplung mit Acroleindialkylacetal bzw. Acrylsäureester unterzogen und anschließend zum Alkohol reduziert. Dabei können Doppelbindung und Carbonylgruppe in beliebiger Reihenfolge reduziert werden. In den Beispielen werden Metallhydride für die Reduktion der Carbonylgruppe und eine Hydrierung der Doppelbindung mit Wasserstoff in Gegenwart von Pd/C als Katalysator als bevorzugt angegeben. Nachteile dieser Vorgehensweise im Vergleich zum hier offen gelegten Verfahren sind der Einsatz von kostspieligen Pd-Katalysatoren für die Heck-Kupplung sowie die Verwendung von sicherheitstechnsich problematischen Metallhydriden für die Reduktion der Carbonylgruppe.

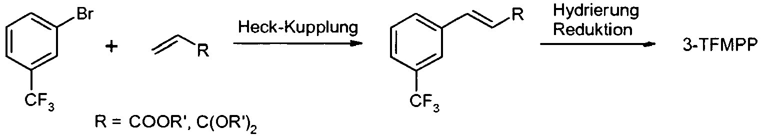

X. Wang et al. beschreiben in einer kurzen Fußnote in Tetrahedron Letters 2004, 45, 8355-8358 einen anderen möglichen Zugang zu 3-TFMPP durch Hydrierung der entsprechenden CF₃-substituierten Zimtsäure und anschließende Reduktion der Säure zum Alkohol mit LiAlH₄.

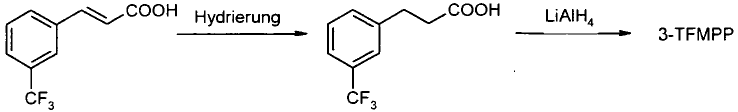

Weiterhin beschreiben Y.-Q. Wu, in J. Med. Chem. 2002, 45 (16), 3549-3557 die Synthese von 3-(4-Trifluormethylphenyl)-propan-1-ol (4-TFMPP) durch Wittig-Reaktion von 4-Trifluormethylbenzaldehyd mit PPh₃CHCOOCH₃, anschließender Doppelbindungshydrierung (H₂, Pd/C) und Ester-Reduktion mit LiAlH₄.

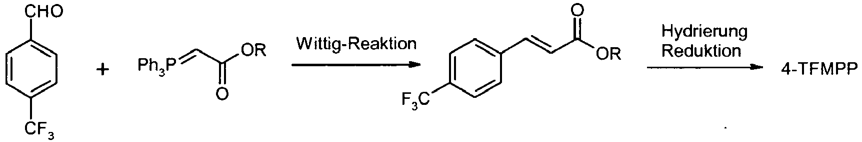

N. J. Green et al., Bioorg. Med. Chem 2003, 11 (13), 2991-3014 beschreibt die Esterkondensation von 3-Trifluormethylbenzoesäuremethylester mit Methylacetat in Gegenwart von Natriumhydrid. Diese Esterkondensation liefert jedoch lediglich eine Ausbeute von 78 %.

Die selektive Hydrierung eines β-Ketoesters zu den entsprechenden gesättigten Estern wird durch K. Hattori et al. in Tetrahedron 2001, 57, 4817-4824 oder von E.J. McWhorter in J. Org. Chem. 1972, 37(23), 3687-3691 mittels Pd/C-Katalysatoren beschrieben.

K. Kindler, Arch. Pharm. 1933, 271, 431-439 beschreiben, dass bei der Hydrierung eines β-Ketoesters zu den entsprechenden Estern der Anteil des eingesetzten Palladiums verringert werden kann, wenn in Gegenwart von sauren Trägermaterial für den Katalysator oder in Gegenwart von Säuren oder sauren Ionentauschern gearbeitet wird.

Die Reduktion eines Esters zu dem entsprechenden Alkohol in Gegenwart von LiAlH₄ wird von Y.-Q. Wu, J. Med. Chem 2002, 45(16), 3549-3557 beschrieben. Die gleiche Umsetzung wird von L.A. Saudan et al. Angew. Chem. 2007, 119(39), 7617-74620 mittels Hydrierung eines Esters zum entsprechenden Alkohol in Gegenwart von homogenen Ru-Katalysatoren beschrieben.

Nachteilig bei den hier beschriebenen Verfahren, oder Teilschritten des erfindungsgemäßen Verfahrens, ist der kostenintensive Katalysatoreinsatz während der Heck-Kupplung, sowie der schwierige Metallhydrideinsatz im technischen Maßstab, der zu einem überhöhten Salzanfall und damit verbundenen sicherheitstechnischen Problemen und zusätzlichen Kosten führt. Des Weiteren verringert der Einsatz der entsprechenden Arylhalogenide aufgrund ihrer korrosiven Eigenschaft den Einsatz des jeweiligen Reaktors.

Die Aufgabe der vorliegenden Erfindung ist daher ein Verfahren zur Herstellung von Phenylalkan-1-olen bereit zu stellen, das den Einsatz kostenintensiver Katalysatoren herabsenkt, somit kostengünstiger ist, den Metallhydrid- sowie den Arylhalogenideinsatz vermeidet und trotzdem in nur wenigen Stufen zum gewünschten Produkt führt.

Diese Aufgabe wird gelöst durch ein Verfahren zur Herstellung von Phenylalkan-1-olen der Formel I 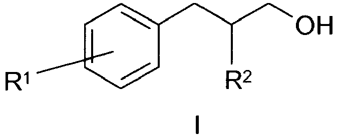 wobei
- R¹: ausgewählt ist aus der Gruppe von Wasserstoff, verzweigten, geradkettigen oder zyklischen Alkylresten mit 1 bis 6 C-Atomen, mit Heteroatomen substituierten geradkettigen, verzweigten oder zyklischen Alkylresten mit 1 bis 6 C-Atomen, substituierten oder unsubstituierten Arylresten, mit Heteroatomen substituierten Arylresten, unsubstituierten Alkoxy- sowie mit Heteroatomen substituierten Alkoxyresten und Halogenresten.
- R²: ausgewählt ist aus der Gruppe von Wasserstoff, verzweigten; geradkettigen oder zyklischen Alkylresten mit 1 bis 6 C-Atomen, substituierten oder unsubstituierten Arylresten.
wobei
a) zuerst eine Verbindung der Formel II 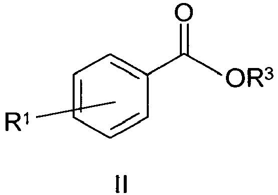 mit
   - R³: einem verzweigten, geradkettigen oder zyklischen Alkylrest mit 1 bis 6 C-Atomen und
   - R¹: mit der obigen Bedeutung
   mit einer Verbindung der Formel III 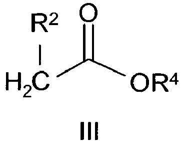
   - mit R⁴: ausgewählt aus der Gruppe von verzweigten, geradkettigen oder zyklischen Alkylrest mit 1 bis 6 C-Atomen und
   - R²: mit der obigen Bedeutung
   in Gegenwart von Alkali- und/oder Erdalkalialkoholaten und einem apolaren Lösungsmittel umgesetzt wird,
b) und der anschließend erhaltende β-Ketoester der Formel IV 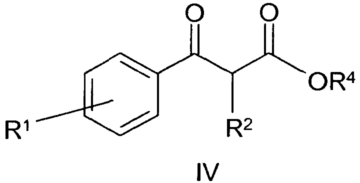 durch selektive Hydrierung mit Palladium als Katalysator und in Gegenwart von Wasserstoff zum entsprechenden Ester der Formel V 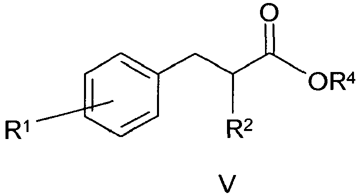 hydriert wird, wobei R¹, R² und R⁴ die obige Bedeutung haben
c) und anschließend im letzen Schritt der Ester der Formel V mittels Wasserstoffzugabe und Katalysator zum Phenylalkan-1-ol der Formel I hydriert wird.

Vorteilhaft ist das erfindungsgemäße Verfahren, wenn der Rest R¹ in dem Phenylalkan-1-ol der Formel I in Position 3 des Phenylringes sitzt.

Vorteilhaft ist das erfindungsgemäße Verfahren, wenn R¹ ausgewählt ist aus der Gruppe von verzweigten oder geradkettigen Alkylgruppen mit 1 bis 3 C-Atomen und mit Heteroatomen substituierten geradkettigen oder verzweigten Alkylgruppen mit 1 bis 3 C-Atomen.

Vorteilhaft ist das erfindungsgemäße Verfahren, wenn R² Wasserstoff und R¹ der Trifluormethylgruppe entspricht.

Vorteilhaft ist das erfindungsgemäße Verfahren, wenn das apolare Lösungungsmittel in Stufe a) Toluol oder Xylol oder Cyclohexan ist.

Vorteilhaft ist das erfindungsgemäße Verfahren, wenn in Stufe b) bei einer Temperatur im Bereich von 20 bis 150°C und einem Druck von 1 bis 200 bar hydriert wird.

Vorteilhaft ist das erfindungsgemäße Verfahren, wenn in Stufe b) Pd/C als Katalysator auf einem sauren Trägermaterial oder in Gegenwart von Säuren oder sauren Ionenaustauschern eingesetzt wird.

Vorteilhaft ist das erfindungsgemäße Verfahren, wenn in Stufe c) ein heterogener Kupferkatalysator eingesetzt wird.

Vorteilhaft ist das erfindungsgemäße Verfahren, wenn in Stufe c) bei einem Druck im Bereich von 50 bis 350 bar und einer Temperatur im Bereich von 100 bis 250 °C gearbeitet wird.

Vorteilhaft ist das erfindungsgemäße Verfahren, wenn nach dem Schritt c) das Phenylalkan-1-ol der Formel I durch Destillation gewonnen wird.

Im Schritt a) des erfindungsgemäßen Verfahrens werden Verbindungen der Formel II 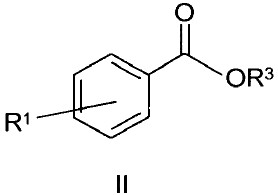 eingesetzt. Die Verbindungen der Formel II sind Ester wobei der Rest R¹ ausgewählt ist aus der Gruppe von Wasserstoff, verzweigten, geradkettigen oder zyklischen Alkylgruppen mit 1 bis 6 C-Atomen, mit Heteroatomen substituierten geradkettigen oder verzweigten Alkylgruppen mit 1 bis 6 C-Atomen, substituierten oder unsubstituierten Arylresten, mit Heteroatomen substituierten Arylresten, unsubstituierten Alkoxy- sowie mit Heteroatomen substituierten Alkoxyresten und Halogenresten. Besonders bevorzugt ist der Rest R¹ ausgewählt aus der Gruppe von Wasserstoff, Methyl, Ethyl, n-Propyl, i-Propyl, n-Butyl, sec-Butyl, tert-Butyl, Trifluormethyl, 1,1,1-Trifluorethyl, Methoxy, Ethoxy, Propyloxy, i-Propyloxy, Phenyl, Tolyl, Anisyl, Chlor, Brom, Fluor. Ganz besonders bevorzugt ist R¹ Methyl oder Trifluormethyl. Insbesondere ganz besonders bevorzugt ist R¹ Trifluormethyl.

Der Rest R¹ kann prinzipiell an jeglicher Stellung des Aromaten sitzen. Bevorzugt sitzt der Rest R¹ in 3 oder 4 Position zur Esterfunktion. Ganz besonders bevorzugt an 3 Position.

Der Rest R³ ein verzweigter, geradkettiger oder zyklischer Alkylrest mit 1 bis 6 C-Atomen. Bevorzugt ist R³ ausgewählt aus der Gruppe von Methyl, Ethyl, n-Propyl, i-Propyl, n-Butyl, sec-Butyl, tert-Butyl, n-Pentyl, Cyclopentyl, n-Hexyl, Cyclohexyl. Bevorzugt sind Methyl und Ethyl. Ganz besonders bevorzugt ist Methyl.

Die Verbindungen der Formel II werden mit Verbindungen der Formel III 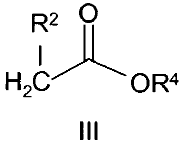 umgesetzt. Bei den Verbindungen der Formel III handelt es sich hierbei ebenfalls um Ester. R² ist hierbei in der Verbindung der Formel III ausgewählt aus der Gruppe von Wasserstoff, verzweigten, geradkettigen oder zyklischen Alkylgruppen mit 1 bis 6 C-Atomen. Besonders bevorzugt ist R² aus der Gruppe von Wasserstoff, Methyl, Ethyl, n-Propyl, i-Propyl, n- Butyl, sec. Butyl, tert-Butyl, n-Pentyl, Cyclopentyl, n-Hexyl, Cyclohexyl. Ganz besonders bevorzugt ist R² Wasserstoff.
Die Umsetzung der Verbindung der Formel II mit den Verbindungen der Formel III erfolgt in Gegenwart von Alkali- bzw. Erdalkalialkoholaten, bevorzugt NaOMe, NaOEt, KOMe, KOEt, KOtBu, besonders bevorzugt KOMe mit einem apolaren Lösungsmittel. Das apolare Lösungsmittel ist dabei ausgewählt aus der Gruppe von Benzol, Toluol, Xylol, Hexan, Cyclohexan, Heptan, Cycloheptan. Besonders bevorzugt sind Toluol, Xylol und Cyclohexan. Ganz besonders bevorzugt sind Toluol und Cyclohexan.

Die Umsetzung der Verbindungen der Formel II mit den Verbindungen der Formel III erfolgt bei Temperaturen im Bereich von 20 bis 140 °C, bevorzugt im Bereich von 50 bis 80 °C.

Aus den Estern der Formel II und III entsteht anschließend der β-Ketoester der Formel IV. Dieser β-Ketoester der Formel IV wird anschließen im Schritt b) selektiv zum Ester der Formel V hydriert. Die Hydrierung des Schrittes b) erfolgt dabei in Gegenwart von Wasserstoff und Palladium sowie einer Säure als Katalysator. Bevorzugt wird diese Hydrierung in Schritt b) bei Temperaturen im Bereich von 20 bis 150 °C, besonders bevorzugt im Bereich von 20 bis 100 °C und einem Wasserstoffdruck im Bereich von 1 bis 200 bar, bevorzugt im Bereich von 1 bis 50 bar durchgeführt. Als Katalysator wird Palladium (0) auf einem Träger verwendet. Als Träger kommen in Frage Aktivkohle, Aluminiumoxid, Siliziumoxid, Titanoxid, Magnesiumoxid, Lanthanoxid, Zinkoxid, Manganoxid, Zirkonoxid, Eisenoxid, Zeolithe und Tone. Besonders bevorzugt ist die Verwendung von Aktivkohle als Träger. Besonders bevorzugt läuft die selektive Hydrierung in Gegenwart eines sauren Trägermaterials für den Katalysator oder aber in Gegenwart von Säuren oder sauren Ionentauschern ab. Als saures Trägermaterial sind Trägermaterialien zu verstehen, die ausgewählt sind aus der Gruppe von Aktivkohle, Aluminiumoxid, Siliziumoxid, Lanthanoxid, Zeolithe und Tone und entsprechend saure Eigenschaften aufweisen. Die Säuren und sauren Ionenaustauscher in deren Gegenwart die Hydrierung stattfinden kann sind, ausgewählt aus der Gruppe von Salzsäure, Schwefelsäure, Phosphorsäure, Wolframatophosphorsäure, Molybdatophosphorsäure, sowie stark sauren Kationentauschern.

In Schritt c) wird der aus Schritt b) entstandene Ester der Formel V anschließend in Gegenwart von Wasserstoff und einem Katalysator zu dem entsprechenden Phenylalkan-1-ol der Formel I hydriert.
Schritt c) wird bevorzugt bei Temperaturen im Bereich von 100 bis 250 °C, besonders bevorzugt im Bereich von 120 bis 200 °C und einem Druck im Bereich von 50 bis 350 bar, besonders bevorzugt im Bereich von 100 bis 250 bar durchgeführt.

Als Katalysator ist jeder Katalysator einsetzbar, der sowohl batchweise als auch kontinuierlich den Ester zum Alkohol hydrieren kann. Bevorzugt ist der Katalysator ausgewählt aus der Gruppe von homogenen Ru-Katalysatoren und heterogenen kupferhaltigen bzw. nickelhaltigen Katalysatoren. Besonders bevorzugt ist ein Katalysator enthaltend CuO/Cu/La₂O₃/Al₂O₃.

Das so erhaltene Phenylalkan-1-ol der Formel 1 kann durch die dem Fachmann bekannten Reinigungsverfahren anschließend aufgereinigt werden. Solche Reinigungsverfahren sind ausgewählt aus der Gruppe von Kristallisation, Destillation, Sublimation, Zentrifugieren und Chromatographie. Besonders bevorzugt ist die Destillation.

Die besonders bevorzugte Verbindung der Phenylalkan-1-ole der Formel I ist 3-(3'-Trifluormethylphenyl)propan-1-ol (3-TFMPP).

### Beispiele

### Synthese von 3-Oxo-3-(3'-trifluormethylphenyl)-propionsäuremethylester

### Beispiel 1:

920 g (4,20 mol) ~32%ige KOMe-Lösung werden vorgelegt und auf 68°C erhitzt. Bei Normaldruck und 85-139°C Innentemperatur wird unter kontinuierlicher Zudosierung von 1988 g iso-Xylol Methanol abdestilliert (Lösungsmitteltausch). Wenn eine Übergangstemperatur von 138°C erreicht ist, wird die Innentemperatur auf 80°C abgesenkt und 321,6 g (1,56 mol) 3-Trifluormethylbenzoesäuremethylester zugegeben. Anschließend werden 186,0 g (2,51 mol) Methylacetat über 3 h bei 78°C zudosiert. Nach 2 h Nachrühren bei 78°C werden weitere 321,6 g (4,34 mol) Methylacetat über 2 h zudosiert. Nach Beendigung der Zugabe wird weitere 4 h bei 78°C nachgerührt und dann auf Raumtemperatur abgekühlt.
Durch Zudosierung von 335 g (3,90 mol) einer 70%igen methanolischen Essigsäurelösung wird ein pH-Wert von 7-8 eingestellt. Die entstandene Suspension wird durch Zugabe von 1047 g Wasser aufgelöst und anschließend werden die Phasen getrennt. Die organische Phase wird mit 400 g ges. NaCl - Lösung gewaschen, über Natriumsulfat getrocknet, eingeengt und der Rückstand (74 g) mittels HPLC analysiert.
U_{Benzoesäureester} = 93 %
A_{Ketoester} = 88 %
S_{Ketoester} = 95 %

### Beispiel 2:

1380 g (6,30 mol) 32%ige KOMe-Lösung werden vorgelegt und auf 80°C erhitzt. Bei Normaldruck und 85-139°C Innentemperatur wird unter kontinuierlicher Zudosierung von 1931 g iso-Xylol Methanol abdestilliert (Lösungsmitteltausch). Wenn eine Übergangstemperatur von 138°C erreicht ist, wird die Innentemperatur auf 80°C abgesenkt und 482,4 g (2,36 mol) 3-Trifluormethylbenzoesäuremethylester zugegeben. Anschließend werden 764,2 g (10,31 mol) Methylacetat über 6 h bei 80°C zudosiert. Nach Beendigung der Zugabe wird weitere 6 h bei 80°C nachgerührt und dann auf Raumtemperatur abgekühlt.
Durch Zudosierung von 356 g (5,92 mol) Essigsäure wird ein pH-Wert von 7-8 eingestellt. Anschließend werden 1420 g Wasser zugegeben und die Phasen getrennt. Die organische Phase wird eingeengt und der Rückstand (562 g) mittels HPLC analysiert.
U_{Benzoesäureester} = 97 %
A_{Ketoester} = 90 %
S_{Ketoester} = 93 %

### Synthese von 3-(3'-Trifluormethylphenyl)-propionsäuremethylester

### Beispiel 3: Verwendung von Pd/C und Amberlyst 15

In einem 9 Liter-Autoklaven wurden 955 g 3-Oxo-3-(3'-trifluormethylphenyl)-propionsäuremethylester (Reinheit 85%) in 4800 ml Methanol gelöst und mit 30,0 g eines Pd/C-Katalysators (5% Pd auf Kohle, Wasseranteil 50%) sowie 24,0 g des sauren Ionentauschers Amberlyst 15 versetzt. Nach Verschließen des Autoklaven wurde auf 60°C aufgeheizt und 10 bar Wasserstoff aufgepresst, wobei der Wasserstoff nach Verbrauch nachdosiert wurde. Nach 48 Stunden wurde der Autoklav nach Abkühlen entspannt und entleert, wobei eine Reaktionslösung enthaltend 727 g 3-(3'-Trifluormethylphenyl)-propionsäuremethylester anfiel, entsprechend einer Ausbeute von 95%.

### Beispiel 4: Verwendung von Pd/C und Amberlyst 39

In einem 9 Liter-Autoklaven wurden 740 g 3-Oxo-3-(3'-trifluormethylphenyl)-propionsäuremethylester (Reinheit 85%) in 2600 ml Methanol gelöst und mit 18,5 g eines Pd/C-Katalysators (5% Pd auf Kohle, Wasseranteil 50%) sowie 37,0 g des sauren Ionentauschers Amberlyst 39 versetzt. Nach Verschließen des Autoklaven wurde auf 60°C aufgeheizt und 10 bar Wasserstoff aufgepresst, wobei der Wasserstoff nach Verbrauch nachdosiert wurde. Nach 48 Stunden wurde der Autoklav nach Abkühlen entspannt und entleert, wobei eine Reaktionslösung enthaltend 649 g 3-(3'-Trifluormethylphenyl)-propionsäuremethylester anfiel, entsprechend einer Ausbeute von 93%.

### Beispiel5: Verwendung von Pd/C und HCl

In einem 300 ml Autoklaven wurden 20 g 3-Oxo-3-(3'-trifluormethylphenyl)-propionsäuremethylester (Reinheit 85%) in 100 ml Methanol gelöst und mit 1,0 g eines Pd/C-Katalysators (5% Pd auf Kohle, Wasseranteil 50%) sowie 0,5 ml Salzsäure (32%ig) 15 versetzt. Nach Verschließen des Autoklaven wurde auf 60°C aufgeheizt und 10 bar Wasserstoff aufgepresst, wobei der Wasserstoff nach Verbrauch nachdosiert wurde. Nach 24 Stunden wurde der Autoklav nach Abkühlen entspannt und entleert, wobei eine Reaktionslösung enthaltend 14,3 g 3-(3'-Trifluormethylphenyl)-propionsäuremethylester anfiel, entsprechend einer Ausbeute von 89%.

### Beispiel 6: Verwendung von Pd/C

In einem 300 ml Autoklaven wurden 20 g 3-Oxo-3-(3'-trifluormethylphenyl)-propionsäuremethylester (Reinheit 85%) in 100 ml Methanol gelöst und mit 3,4 g eines Pd/C-Katalysators (3% Pd auf Kohle, Wasseranteil 50%) versetzt. Nach Verschließen des Autoklaven wurde auf 60°C aufgeheizt und 10 bar Wasserstoff aufgepresst, wobei der Wasserstoff nach Verbrauch nachdosiert wurde. Nach 24 Stunden wurde der Autoklav nach Abkühlen entspannt und entleert, wobei eine Reaktionslösung enthaltend 14,7 g 3-(3'-Trifluormethylphenyl)-propionsäuremethylester anfiel, entsprechend einer Ausbeute von 92%.

### Synthese von 3-(3'-Trifluormethylphenyl)-propan-1-ol (3-TFMPP)

### Beispiel 7:

In einem 50 ml Autoklaven wurden 20 ml des Austrags aus Beispiel 3 (enthaltend 2,5 g 3-(3'-Trifluormethylphenyl)-propionsäuremethylester) mit 2 g eines Katalysators der Zusammensetzung 56% CuO, 15% Cu, 24% Al₂O₃ und 4% La₂O₃ versetzt und für 24 Stunden bei 180°C und 200 bar Wasserstoffdruck gerührt. Nach Abkühlen und Entspannen wurde eine Lösung enthaltend 2,0 g 3-TFMPP ausgebaut, entsprechend einer Ausbeute von 91 %.

### Beispiel 8:

Über einen Reaktor mit 100 ml des Katalysators der Zusammensetzung 56% CuO, 15% Cu, 24% Al₂O₃ und 4% La₂O₃ wurde eine Lösung von 3-(3'-Trifluormethylphenyl)-propionsäuremethylester in Methanol (Gehalt 20 Gew%) mit 40 g/h bei 200 bar Wasserstoffdruck und 160°C zugefahren, wobei Wasserstoff mit 100 Nl/h zudosiert wurde. Im Austrag wurden noch 0,1 Gew% des Edukts neben 17,3 Gew% an 3-(3'-Trifluormethylphenyl)-1-propanol in der Lösung gefunden, entsprechend einem Umsatz >99% und einer Alkohol-Ausbeute von 98%.

## Patentansprüche

1. Verfahren zur Herstellung von Phenylalkan-1-olen der Formel I 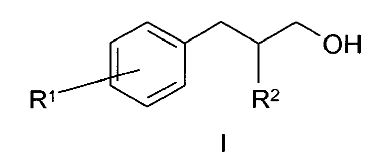 wobei
R¹ ausgewählt ist aus der Gruppe von Wasserstoff, verzweigten, geradkettigen oder zyklischen Alkylresten mit 1 bis 6 C-Atomen, mit Heteroatomen substituierten verzweigten, geradkettigen oder zyklischen Alkylresten mit 1 bis 6 C-Atomen, substituierten oder unsubstituierten Arylresten, mit Heteroatomen substituierten Arylresten, unsubstituierten Alkoxy- sowie mit Heteroatomen substituierten Alkoxyresten und Halogenresten,
R² ausgewählt ist aus der Gruppe von Wasserstoff, verzweigten, geradkettigen oder zyklischen Alkylresten mit 1 bis 6 C-Atomen, substituierten oder unsubstituierten Arylresten,
wobei
a) zuerst eine Verbindung der Formel II 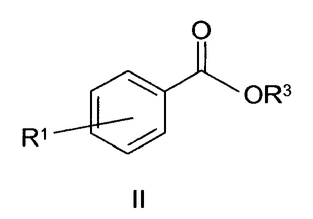
mit R³ einem verzweigten, geradkettigen oder zyklischen Alkylrest mit 1 bis 6 C-Atomen und
R¹ mit der obigen Bedeutung
mit einer Verbindung der Formel III 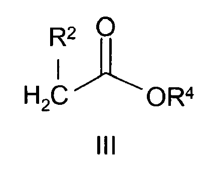
mit R⁴ ausgewählt aus der Gruppe von verzweigten, geradkettigen oder zyklischen Alkylresten mit 1 bis 6 C-Atomen und
R² mit der obigen Bedeutung
in Gegenwart von Alkali- oder Erdalkalialkoholaten, und einem apolaren Lösungsmittel umgesetzt wird,
b) und der anschließend erhaltende β-Ketoester der Formel IV 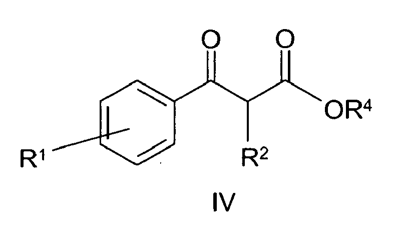 durch selektive Hydrierung mit Palladium als Katalysator und in Gegenwart von Wasserstoff zum entsprechenden Ester der Formel V 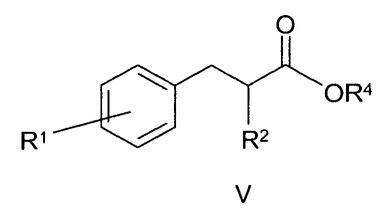 hydriert wird, wobei R¹, R² und R⁴ die obige Bedeutung haben
c) und anschließend im letzen Schritt der Ester der Formel V mittels Wasserstoffzugabe und Katalysator zum Phenylalkan-1-ol der Formel I hydriert wird.

2. Verfahren nach Anspruch 1, wobei der Rest R¹ in dem Phenylalkan-1-ol der Formel I in Position 3 sitzt.

3. Verfahren nach einem der Ansprüche 1 bis 2, wobei R¹ ausgewählt ist aus der Gruppe von verzweigten oder geradkettigen Alkylgruppen mit 1 bis 3 C-Atomen und mit Heteroatomen substituierten verzweigten oder geradkettigen Alkylgruppen mit 1 bis 3 C-Atomen.

4. Verfahren nach einem der Ansprüche 1 bis 3, wobei R² Wasserstoff und R¹ der Trifluormethylgruppe entspricht.

5. Verfahren nach einem der Ansprüche 1 bis 4, wobei das apolare Lösungungsmittel in Stufe a) Toluol oder Xylol oder Cyclohexan ist.

6. Verfahren nach einem der Ansprüche 1 bis 5, wobei in Stufe b) bei einer Temperatur im Bereich von 20 bis 150 °C und einem Druck von 1 bis 200 bar hydriert wird.

7. Verfahren nach einem der Ansprüche 1 bis 6, wobei in Stufe b) Pd/C als Katalysator auf einem sauren Trägermaterial oder in Gegenwart von Säuren oder sauren Ionenaustauschern eingesetzt wird.

8. Verfahren nach einem der Ansprüche 1 bis 7, wobei in Stufe c) ein heterogener Kuperkatalysator eingesetzt wird.

9. Verfahren nach einem der Ansprüche 1 bis 8, wobei in Stufe c) bei einem Druck im Bereich von 100 bis 350 bar und einer Temperatur im Bereich von 100 bis 350 °C gearbeitet wird.

10. Verfahren nach einem der Ansprüche 1 bis 9, wobei nach dem Schritt c) das Phenylalkan-1-ol der Formel I durch Destillation gewonnen wird.

## Claims

1. A process for preparing phenylalkan-1-ols of the formula I 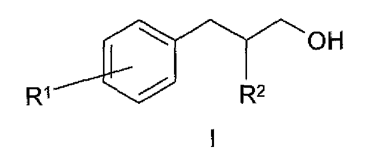 where
R¹ is selected from the group of hydrogen, branched, straight-chain or cyclic alkyl radicals having 1 to 6 C atoms, straight-chain, branched or cyclic alkyl radicals which are substituted by heteroatoms and have 1 to 6 C atoms, substituted or unsubstituted aryl radicals, aryl radicals substituted by heteroatoms, unsubstituted alkoxy radicals, and alkoxy radicals substituted by heteroatoms, and halogen radicals.
R² is selected from the group of hydrogen, branched, straight-chain or cyclic alkyl radicals having 1 to 6 C atoms, substituted or unsubstituted aryl radicals,
where
a) firstly a compound of the formula II 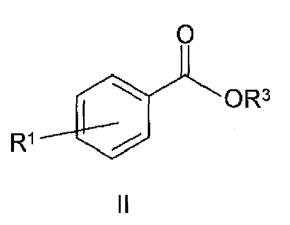
with R³ a branched, straight-chain or cyclic alkyl radical having 1 to 6 C atoms and
R¹ with the above meaning,
is reacted with a compound of the formula III 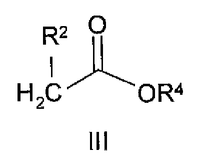
with R⁴ selected from the group of branched, straight-chain or cyclic alkyl radicals having 1 to 6 C atoms and
R² with the above meaning
in the presence of alkali metal and/or alkaline earth metal alcoholates and a nonpolar solvent,
b) and the subsequently obtained β-keto ester of the formula IV 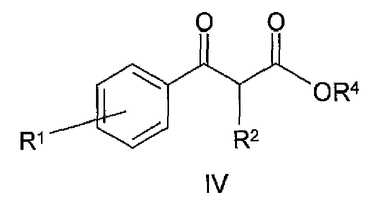 is hydrogenated by selective hydrogenation with palladium as catalyst and in the presence of hydrogen to give the corresponding ester of the formula V 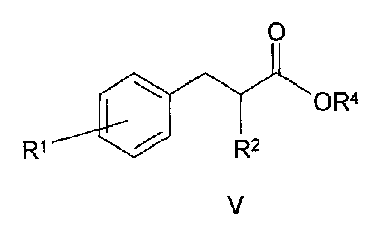 where R¹, R² and R⁴ have the above meaning,
c) and subsequently in the last step the ester of the formula V is hydrogenated by addition of hydrogen and catalyst to the phenylalkan-1-ol of the formula I.

2. The process according to claim 1, where the radical R¹ is located in position 3 in the phenylalkan-1-ol of the formula I.

3. The process according to either of claims 1 to 2, where R¹ is selected from the group of branched or straight-chain alkyl groups having 1 to 3 C atoms and branched or straight-chain alkyl groups substituted by heteroatoms and having 1 to 3 C atoms.

4. The process according to any of claims 1 to 3, where R² corresponds to hydrogen and R¹ corresponds to the trifluoromethyl group.

5. The process according to any of claims 1 to 4, where the nonpolar solvent in stage a) is toluene or xylene or cyclohexane.

6. The process according to any of claims 1 to 5, where the hydrogenation in stage b) is carried out at a temperature in the range from 20 to 150°C under a pressure of from 1 to 200 bar.

7. The process according to any of claims 1 to 6, where the catalyst employed in stage b) is Pd/C on an acidic support material or in the presence of acids or acidic ion exchangers.

8. The process according to any of claims 1 to 7, where a heterogeneous copper catalyst is employed in stage c).

9. The process according to any of claims 1 to 8, where stage c) is carried out under a pressure in the range from 100 to 350 bar and at a temperature in the range from 100 to 350°C.

10. The process according to any of claims 1 to 9, where the phenylalkan-1-ol of the formula I is obtained by distillation after step c).

## Revendications

1. Procédé de fabrication de phénylalcan-1-ols de formule I 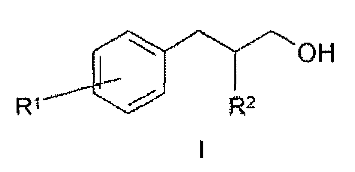 dans laquelle
R¹ est choisi dans le groupe constitué par l'hydrogène, les radicaux alkyle ramifiés, linéaires ou cycliques de 1 à 6 atomes C, les radicaux alkyle ramifiés, linéaires ou cycliques de 1 à 6 atomes C substitués avec des hétéroatomes, les radicaux aryle substitués ou non substitués, les radicaux aryle substitués avec des hétéroatomes, les radicaux alcoxy non substitués et les radicaux alcoxy substitués avec des hétéroatomes, et les radicaux halogène,
R² est choisi dans le groupe constitué par l'hydrogène, les radicaux alkyle ramifiés, linéaires ou cycliques de 1 à 6 atomes C, les radicaux aryle substitués ou non substitués,
dans lequel
a) un composé de formule II 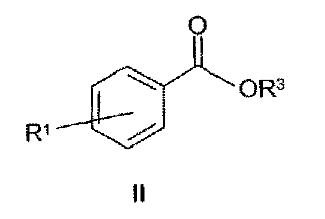 dans laquelle R³ représente un radical alkyle ramifié, linéaire ou cyclique de 1 à 6 atomes C, et
R¹ a la signification précédente,
est tout d'abord mis en réaction avec un composé de formule III 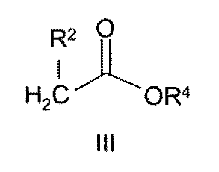 dans laquelle R⁴ est choisi dans le groupe constitué par les radicaux alkyle ramifiés, linéaires ou cycliques de 1 à 6 atomes C, et
R² a la signification précédente,
en présence d'alcoolates alcalins ou alcalino-terreux et d'un solvant apolaire,
b) et le β-cétoester de formule IV ensuite obtenu 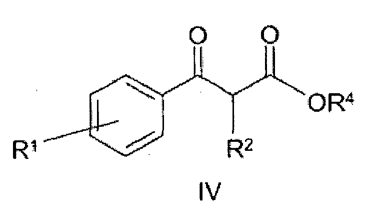 est hydrogéné par hydrogénation sélective avec du palladium en tant que catalyseur et en présence d'hydrogène en l'ester de formule V correspondant 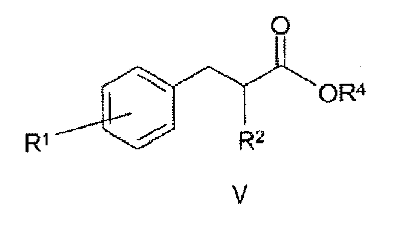 dans laquelle R¹, R² et R⁴ ont la signification précédente,
c) puis, lors de la dernière étape, l'ester de formule V est hydrogéné par ajout d'hydrogène et d'un catalyseur en le phénylalcan-1-ol de formule I.

2. Procédé selon la revendication 1, dans lequel le radical R¹ dans le phénylalcan-1-ol de formule I est en position 3.

3. Procédé selon l'une quelconque des revendications 1 à 2, dans lequel R¹ est choisi dans le groupe constitué par les groupes alkyle ramifiés ou linéaires de 1 à 3 atomes C et les groupes alkyle ramifiés ou linéaires de 1 à 3 atomes C substitués avec des hétéroatomes.

4. Procédé selon l'une quelconque des revendications 1 à 3, dans lequel R² correspond à l'hydrogène et R¹ au groupe trifluorométhyle.

5. Procédé selon l'une quelconque des revendications 1 à 4, dans lequel le solvant apolaire à l'étape a) est le toluène ou le xylène ou le cyclohexane.

6. Procédé selon l'une quelconque des revendications 1 à 5, dans lequel l'hydrogénation à l'étape b) est réalisée à une température dans la plage allant de 20 à 150 °C et à une pression de 1 à 200 bar.

7. Procédé selon l'une quelconque des revendications 1 à 6, dans lequel, à l'étape b), Pd/C est utilisé en tant que catalyseur sur un matériau support acide ou en présence d'acides ou d'échangeurs d'ions acides.

8. Procédé selon l'une quelconque des revendications 1 à 7, dans lequel un catalyseur hétérogène à base de cuivre est utilisé à l'étape c).

9. Procédé selon l'une quelconque des revendications 1 à 8, dans lequel l'étape c) est réalisée à une pression dans la plage allant de 100 à 350 bar et à une température dans la plage allant de 100 à 350 °C.

10. Procédé selon l'une quelconque des revendications 1 à 9, dans lequel le phénylalcan-1-ol de formule I est obtenu par distillation après l'étape c).
